# EUROPEAN PATENT APPLICATION

(11) **EP 4 339 598 A1**
(43) Date of publication of application: **20.03.2024**
(21) Application number: 22315209.1
(22) Date of filing: 13.09.2022
(51) Int. Cl.: G01N 21/78, G01N 33/493, G01N 21/77

(54) **OPTICAL MASK FOR TEST STRIP**

(71) Applicant: Withings, 92130 Issy-les-Moulineaux (FR)
(72) Inventor: Barakat, Christelle, 92130 Issy-les-Moulineaux (FR); Lefebvre, Laura, 92130 Issy-les-Moulineaux (FR); Chan, Tung Chi, 92130 Issy-les-Moulineaux (FR)
(74) Representative: Withings IP

(57) **Abstract**

The invention concerns a test strip (700a, 700b, 700c) for an optical analysis a substance. The test strip comprises a detection region (704) comprising a reagent to react directly or indirectly with the substance and an optical mask (708a, 708b, 708c) including a transparent portion (710a) facing at least a part of the detection region (604, 704) and an opaque portion (712a) adjacent to the transparent portion (712a).

## Description

The present disclosure relates to test strip for optical analysis of a substance such as urine.

### State of the art

Document WO2021/175909 discloses a point-of-care device for urine analysis. The device is to be lodged in a toilet (more precisely on a surface of the toilet bowl) and collects sample of a urine stream before performing an optical analysis. The device comprises a station and a cartridge, which is also called a rotatable support, and which may be removed and replaced from the station. The cartridge contains urinary test strips, that is to say strips coated or impregnated with a reagent that reacts with urine.

The test is an optical test, run by a light source and an optical sensor. Accurate data and the detection of a change in the physical/chemical property of the test strip is hard to achieve.

### Summary of the invention

The inventors have noticed that one difficulty of the optical analysis was due to light leaks that occurs when light goes where it should not go and then reach the optical sensor. Therefore, the present disclosure is aimed at presenting a solution which does not present any of the explained limitation.

The present disclosure relates to embodiments of a test trip including an optical mask. The test strip may be mounted in a cartridge usable in a station for urine analysis that can be installed on a surface of a toilet bowl. The cartridge mounted on the station will be referred to as an analysis device. The station comprises a light source and an optical sensor to carry out an optical analysis on the test strip.

The invention is defined in the appended claims.

In particular, the disclosure relates to a test strip for an optical analysis of a substance is provided. The test strip comprises a detection region comprising a reagent to react directly or indirectly with the substance. In addition, the test strip comprises an optical mask including an opaque portion and a transparent portion. The transparent portion faces at least a part of the detection region, and the opaque portion is adjacent to the transparent portion.

Thanks to the optical mask, the detection region may be optically analyzed using light coming from the detection region and not by light coming from another component of the test strip. The sensitivity of the optical sensor may be improved and the accuracy of the data is improved as well.

The transparent portion is designed to not interfere with the optical analysis. In particular, in one embodiment, the transparent portion is defined as an area of the optical mask, which is not the opaque portion, i.e. the transparent portion is defined by a negative space of the opaque portion.

In one implementation, the transparent portion is smaller than the detection region, in particular smaller along the longitudinal direction. This ensures that the majority of light reaching the optical sensor has gone through the detection region, as light coming from the optical mask may be diffracted in different directions.

In one embodiment, the test strip extends along a longitudinal direction and the opaque portion extends on at least one side of the transparent region along the longitudinal direction. In particular, the opaque portion may be positioned in the vicinity of the detection region. By vicinity, it is meant that the opaque portion extends on at least one side of the detection region along the longitudinal direction.

In one embodiment, the test strip comprises a migration pathway in contact with the detection region and configured to receive and migrate the substance. The optical mask covers at least partially the migration pathway.

In one embodiment, the optical mask covers more than 90% of the migration pathway.

Depending on the nature of the analysis, the detection region may include at least two subregions spaced apart from each other, notably by the migration pathway. Those two subregions typically include a test subregion and a control subregion. The opaque portion may extend between the two subregions and notably cover the migration pathway therebetween.

For structural reasons, the test strip usually includes a backing supporting the detection region.

In one implementation, the optical mask is positioned, along a direction transversal to the longitudinal axis, between the detection region (and the migration pathway) and the backing. This implementation is efficiently for an analysis in transmission and is easy to manufacture.

In one implementation, the backing is positioned between the optical mask and the detection region.

In one implementation, the optical mask is provided on a face on the backing. This allows to easily manufacture the optical mask, with minimum adaptation of the test strip. For example, the optical mask is printed on the backing or made of tape attached to one face of the backing.

In one implementation, the detection region is positioned between the optical mask and the backing (i.e., the optical mask is on top of the detection region and the migration pathway). This is particularly appropriate for colorimetric test strip.

The transparent portion may have a rectangular shape extending transversally to the longitudinal axis.

A length of the test strip may be less than 20mm, and/or a width of the test strip may be less than 5mm. For example, the length may be less than 15mm (e.g., around 12mm). Because of the small size of the test strip, which allows to have a urine analysis device with numerous test strips stored therein, the optical challenges are greater than regular sizes of test strip (which are usually a few centimeter-long). The optical mask brings optical precision to a miniaturized version of the test strips.

For manufacturing reasons, the transparent portion of the optical mask extends along a whole width of the test strip.

In particular, the disclosure also relates to a cartridge, comprising a plurality of chambers arranged next to one another in a right circular cylinder shape of at least 80% of a circle with a diameter comprised between 3 and 10 cm, wherein each chambers comprises a test strip as described above.

The plurality of chambers may include at least 10 chambers, in particular, more than 20 chambers, for example between 20 and 100, or between 50 and 100 closed chambers, or between 60 and 90 chambers.

In particular, the disclosure also relates to a urine analysis device for urine analysis comprising a cartridge as disclosed above and a station. The station is configured to be positioned on a wall of a toilet bowl and comprises a case with an annular housing in which the cartridge is at least partially received and is rotatably mounted therein around a rotation axis. The station further comprises an injector, configured to inject urine on the test strip, and an analyzer configured to emit and receive light, and to detect a change of a property (e.g., a color) of the detection zone of the test strip. The analyzer may include a light source configured to emit light towards the test strip and a light sensor configured to receive light from the test strip.

In particular, the disclosure also relates to a process to manufacture a test strip as described above. The process comprises: providing a backing sheet, generating an optical mask by generating on a side face of the backing sheet an opaque zone and a transparent zone, cutting the backing sheet. This process is cost-efficient and allows to mass-produce the test strips.

### Drawings

For a better understanding of the various embodiments described in the enclosed reference is made to drawing introduced here below:
- [FIG. 1]: [Figure 1] shows the overall setup of an analysis device for urine analysis according to an embodiment, as installed on a surface of a toilet bowl;
- [FIG. 2]: [Figure 2] shows an exploded view of an embodiment of an analysis device, in which the station and the cartridge are visible;
- [FIG. 3]: [Figure 3] shows a detailed view of a cartridge according to an embodiment;
- [FIG. 4]: [Figure 4] shows a sectional view of an embodiment of a cartridge and a station, at the location of an optical analyzer of the station;
- [FIG. 5]: [Figure 5] shows two variations of a test strip without an optical mask;
- [FIG. 6]: [Figure 6] shows a schematized variation of a test strip without an optical mask;
- [FIG. 7]: [Figure 7] shows a side view different schematized embodiments of a test strip with an optical mask;
- [FIG. 8]: [Figure 8] shows a perspective view of schematized embodiments of a test strip with an optical mask, in a perspective view;
- [FIG. 9]: [Figure 9] shows a side view of schematized variants of a test strip with an optical mask;
- [FIG. 10]: [Figure 10] shows a schematic view of an arrangement similar to that of [Fig. 4], in which the test strip comprise an optical mask and the light beams are illustrated as dashed lines;
- [FIG. 11]: [Figure 11] shows a process to manufacture a test strip according to the invention.

### Detailed description

The present description introduces different examples of a cartridge usable with a station as disclosed in document WO2021/1759O9 and WO2021/175944 (publication numbers), hereafter referred to as WO'909 and WO'944. Variations of the stations are presented in any of FR2109383, FR2109384, FR2109391, FR2109392 (filing numbers).

The next paragraphs explain the overall principal of a device for urine analysis, but all the details of WO'909 and WO'933 (and also any of the above-mentioned French filings) are applicable.

### OVERALL DESCRIPTION OF THE STATION AND THE CARTRIDGE

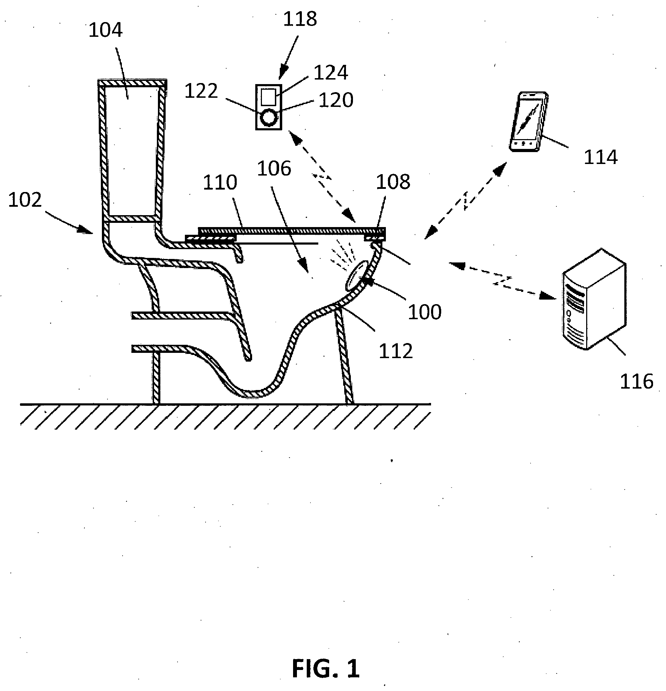

[FIG. 1] schematically illustrates an analysis device 100 (referred to as the "device 100") for urine analysis as set up in toilets 102. Toilets 102 usually comprise a water tank 104, a bowl 106, a seat 108 and a seat cover 110. The analysis device 100 is arranged in a removable manner in the toilets 102. For example, the analysis device 100 may be easily removed from the toilets to replace a cartridge and then arranged again in the toilets 102. The analysis device 100 is arranged on an internal wall 112 of the bowl 106 of the toilets. The analysis device 100 is placed so that it is usually under a urine stream from a user, such that when a user urinates (typically in a seated position), urine contacts with the analysis device 100. The analysis device 100 may communicate remotely with a remote entity, such as smartphone 114 or a server 116.

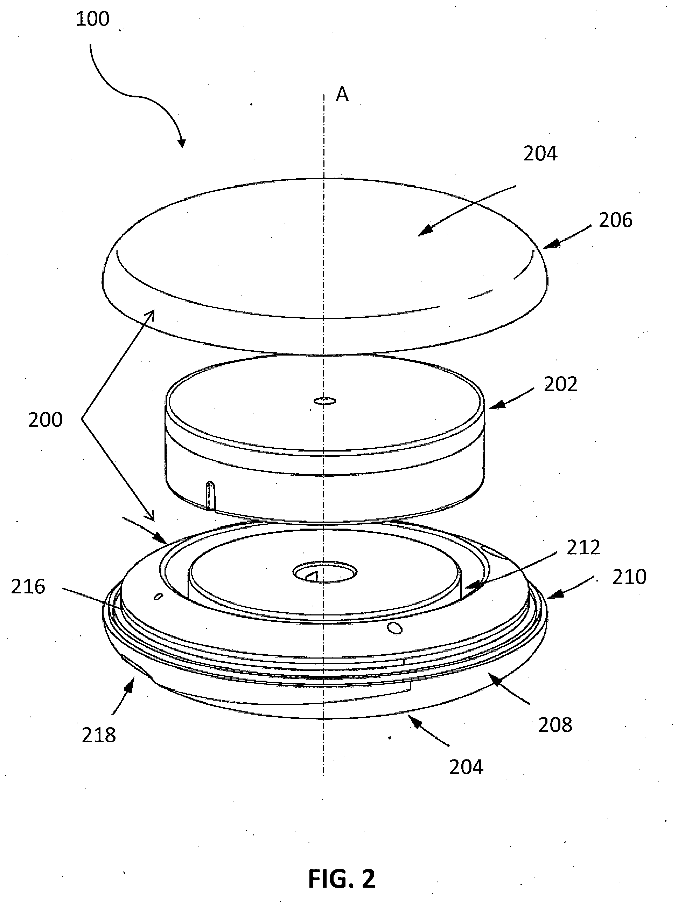

As illustrated in more detail on [FIG. 2], the analysis device 100 comprises a station 200 and a cartridge 202, mounted in a removable manner from the station 200. Station 200 may comprise a case 204 which may include two shells 206, 208. Case 204 lodges therein a urine testing assembly. Station 200 comprises an annular or ring-shaped housing 212, located inside the case 204, arranged around a rotation axis A. The annular housing 212 is configured to receive at least partially the cartridge 202 mounted in a rotatable manner around the rotation axis A (once in position in the annular housing 212). The cartridge 202 comprises a plurality of test supports which each comprise at least one urine reagent, for instance a dry reagent, the plurality of test supports being arranged along a circle or a circular arc around the rotation axis A. In an embodiment, the test supports are test strips. The test supports may be enclosed, for example individually enclosed, in a chamber.

The annular housing 212 typically extends around 360° and forms a groove configured to receive at least partially the cartridge 202.

Station 200 comprises a collection opening 218, located for example on shell 208. The collection opening 218 collects urine flowing on the surface of the housing 204. A drain open-. ing (not illustrated) is also included to drain the liquid out of the device 100.

The housing 204 may have a diameter, in a direction perpendicular to the rotation axis A, comprised between 50 and 150 mm.

The test assembly comprises a pump, an injector and an analyzer. The pump sucks urine from the collection opening 218, then the injector injects the urine on one or more test supports of the cartridge and the analyzer obtains some values of properties (e.g., physical/chemical properties, such as the color) of the test supports after it has contacted the urine. In one embodiment, the analyzer is an optical analyzer configured to analyze optical properties of the test support. The injector and the cartridge may move relatively to each other so that the injector can open (e.g., pierce) the chamber, for example with a needle or needle-like device.

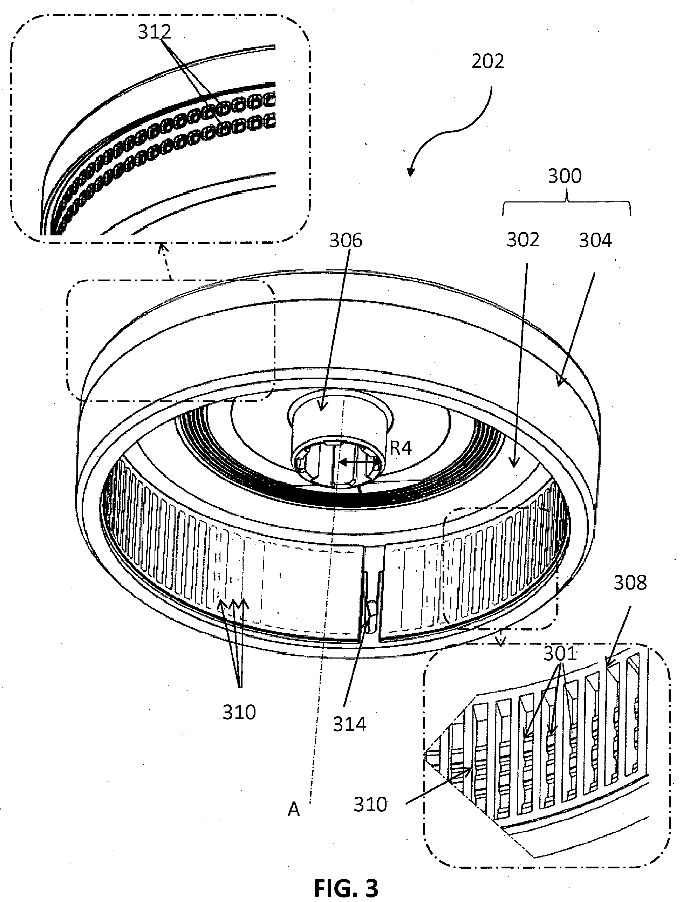

[FIG. 3] shows an exploded view of the cartridge 202. The cartridge 202 comprises test supports 301 configured to receive urine from the injector. The test support contains a urine reagent that reacts in a specific way when in contact with urine. The cartridge 202 comprises a rotatable support 300, configured to be driven in rotation by the station 200. In normal use of the cartridge 202 and the device 100, the test supports 301 remain attached to the rotatable support and do not move with respect to the latter.

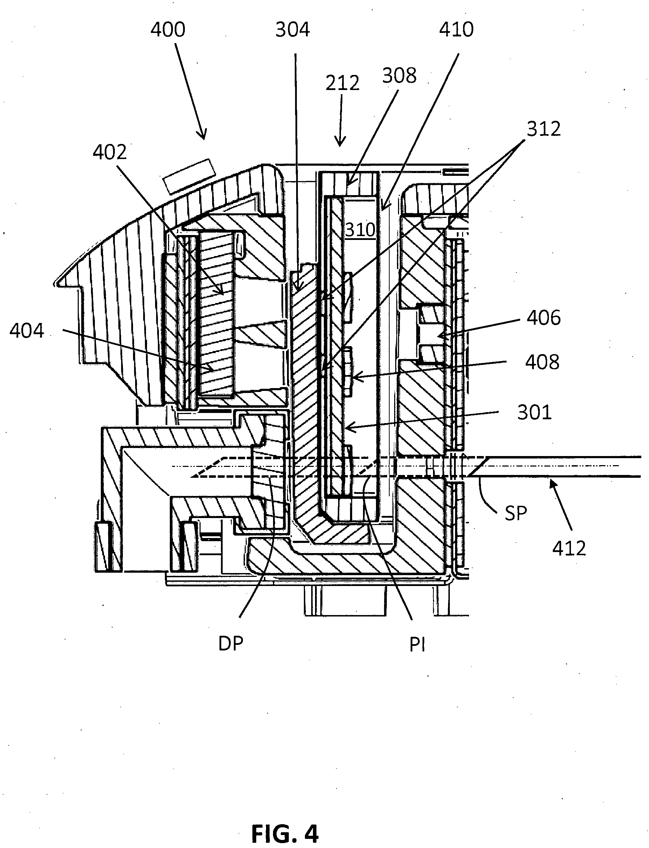

In an embodiment, the rotatable support 300 has a hollow cylinder shape extending annularly around an axis which is, when the cartridge 202 is mounted in the station 200, the rotation axis A. The test support 301 may be a test strip. The rotatable support 300 may comprise an annular portion 302 and a cylindrical portion 304, which extends from an outermost radial extremity of the annular portion 302. The cylindrical portion 304, when in use, is lodged inside the annular chamber 212. The test supports 301 are positioned along the cylindrical portion 304, so that they can be selectively and/or successively scrolled in front of the injector and the analyzer. For instance, the test supports 301 are part of a holder 308, which comprises several chambers 310, separated from each other along a perimeter around the axis A. To allow light to go through, the holder 308 includes at least one aperture 312 per chamber 310 (represented in the upper left zoom where the rotatable support is shown as transparent). The chambers 310 are all at an equal distance of the rotation axis A, so that the injector can selectively inject urine after the desired chamber is positioned at a desired location facing the injector. The injector may translate towards the chamber 310 and pierce a lid closing the chamber 310 (visible on [FIG. 4]). A drain opening 314 is provided in the rotatable support 300 to allow evacuating urine from the injector to the outside of the device 100.

The annular portion 302 of the rotatable support 300 remains outside of the annular chamber 212 to strengthen the cylindrical portion and/or to drive in rotation the cartridge 202. To that end, the annular portion 302 may include a mechanical coupling 306, which cooperates with a shaft of the station 200.

The dimensions relative to the cartridge 202 are disclosed in WO'909, WO'933 and the above-mentioned French applications. The maximum dimension of the device 100 transversal to the rotational axis A is less than 15 cm, even less than 10 cm. The maximum dimension of the device along the rotation axis A is less than 5 cm.

[FIG. 4] shows in more detail the interaction between the cartridge 202 and the station 200 when or after the injector is activated. The analyzer 400 comprises at least one light source 402, 404 (e.g., two) and at least one optical sensor 406. Light travels from the light source 402, 404 to the optical sensor 406 while passing through the cartridge 202 and in particular the cylindrical portion 304, the aperture 312 of the holder 308, the test support 301 and thus the reagent on a reagent zone 408. The injector includes an injection end 412 (for example a needle), which can be moved between several positions, which are represented in dash lines in [FIG. 4]. In a standby position SP, the injection end 412 is outside the cartridge 202 (in an innermost position), so that the cartridge 202 can rotate freely in the annular housing 212; in an injection position IP, the injection end 412 has pierced the lid 410 to access the inside of the chamber 310 and may inject some urine on the test support 301; in the drain position DP, the injection end 412 is able to evacuate urine through the drain opening 314 of the rotatable support 300.

In position SP, the injector is located radially inward the annular chamber. This allows to maximize the radius of the annular chamber while minimizing the size of the station 200.

### OVERALL DESCRIPTION OF THE TEST STRIP

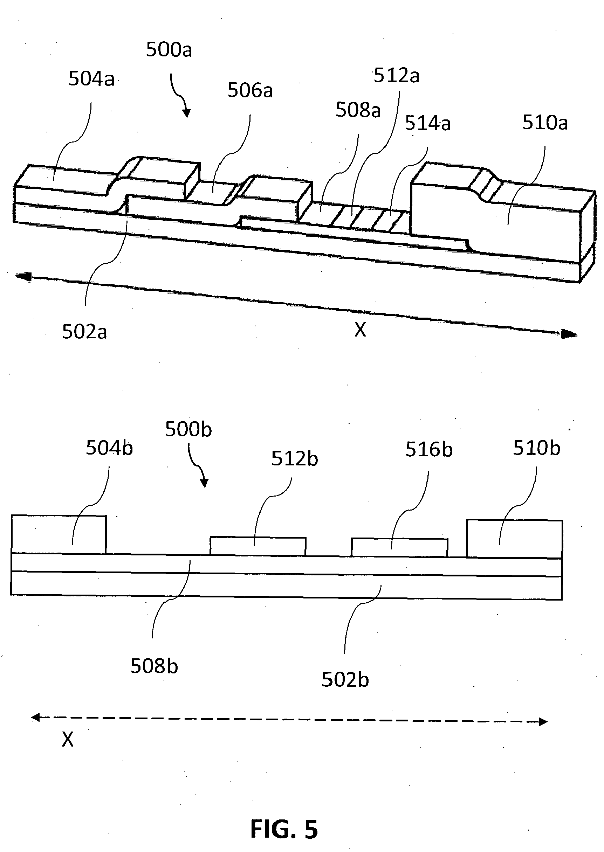

As shown in [Fig. 3], [Fig. 4] and [Fig. 5], the test support may be a test strip. In one embodiment, the test strips have a generally rectangular shape, with a width between 0.5mm and 3mm and a length between 10mm and 20mm, even between 10mm and 15mm, with a thickness that varies along the strip depending on its components. The test strip comprises different components and materials that absorb and/or react to urine.

[Fig. 5] illustrates a test strip 500a, which is a lateral or vertical flow immunoassay strip. The test strip extends notably along a longitudinal direction X. The test strip 500a comprises a backing 502a (e.g., in plastic) which carries: a sample pad 504a, in contact with a conjugate pad 506a, in contact with a migration membrane 508a (e.g., in nitrocellulose), in contact with a wicking pad 510a, thus forming a migration pathway for liquid between the sample pad 504a and the wicking pad 510a.

The migratory membrane 508a comprises at least one test line 512a and one control line 514a arranged one after the one, the test line 512a being on the side of the conjugate pad 506a and the control line 514a being on the side of the wicking pad 510a.

When a urine sample is introduced onto the sample pad 504a, urine migrates by capillary action through the conjugate pad 506a, the test line 512a and the control line 514a to the wicking pad 510a. Conjugate pad 506a, test line(s) 512a and control line 514a contain reagents.

Conjugate pad 506a may include detection antibodies sensitive to compounds in the urine. If the compounds are present when the urine sample passes through the conjugate pad 506a, then the antibodies bind to the compounds to form markers. The markers migrate to test line 512a. Test line 512a may include test antibodies. The test antibodies bind with the markers and retain them on the test line 512a where a colored line is formed, and the density of the line varies according to the concentration of markers present. The remaining sample migrates to a control line 514a, which contains control antibodies, to indicate that the sample has passed through the migrating membrane 508a.

For example, the test strips 500a may be an ELISA-type test strips. This type of test strip 500a allows for example detection of the pregnancy hormone hCG in urine. For example, the detection antibody may be a "mouse monoclonal beta hCG", the test antibody may be a "goat polyclonal anti-mouse IgG" and the control antibody may be a "rabbit polyclonal anti-mouse IgG".

[Fig. 5] also illustrates a test strip 500b which is a colorimetric test strip. The test strip 500B comprises a backing 502b which carries: a sample pad 504b, in contact with a migration membrane 508b (e.g., nitrocellulose), in contact with a wicking pad 510b, thereby forming a migration pathway for liquid between the sample pad 504b and the wicking pad 510b.

The migration membrane 508b comprises or carries at least one colorimetric pad 512b and, optionally, a control pad 516b arranged in sequence, with the colorimetric pad 512b on the side of the sample pad 504b and the control pad 516b on the side of the wicking pad 510b. Several colorimetric pads may be included.

When a urine sample is introduced onto the sample pad 504b, urine migrates by capillary action towards the colorimetric pad 512b and the control pad 516b. The colorimetric pad 512b and the control pad 516b contain reagents.

Those examples of test strips are not limiting.

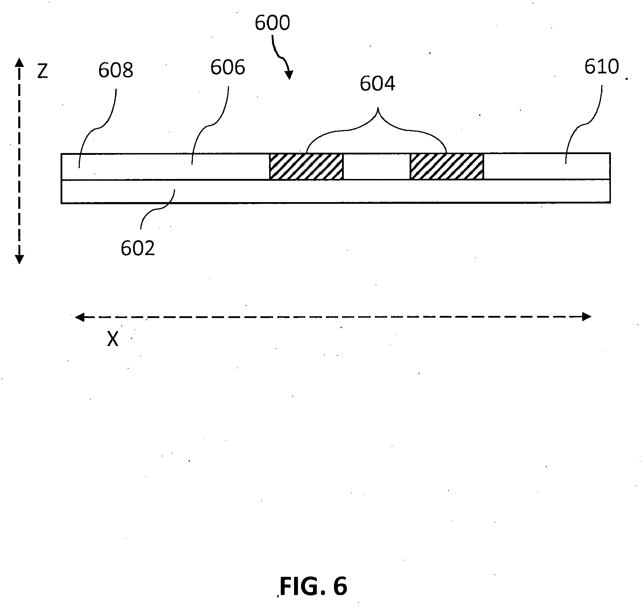

[Fig. 6] illustrates in schematic way a test strip 600. The test strip 600 extends notably along a longitudinal direction X. The test strip 600 presents a length along the longitudinal direction X, a width along a Y direction transversal to the longitudinal X and a thickness along the Z direction, transversal to the X direction and the Y direction. The length is larger than the width and the thickness (for example at least 10 times, hence the name of "strip").

The test strip 600 includes a backing 602 and a detection region 604 (shown in hatched in all the drawings), mounted directly or indirectly on the backing 602. The backing 602 is aimed at rigidifying the test strip. The detection region 604 includes a reagent that reacts directly or indirectly in the presence of the substance of interest (by directly, it is meant that the reagent reacts with the substance of interest and by indirectly, it is meant that the reagent reacts with a compound which has itself reacted with the substance of interest). The test strip 600 may also include a migration pathway 606, configured to transfer urine from a depositing region 608 (e.g., a sample pad) configured to receive the urine sample to a collection region 610 (e.g., a wicking pad) configured to draw urine through the migration pathway 606. The detection region 604 is connected to the migration pathway 606. Alternatively, depending on the type of test strip and the station 200, no migration pathway is required, and the urine sample is deposited directly on the detection region 604.

For the optical analysis, the backing is preferably transparent or at least does not hinder or prevent the optical analysis.

The detection region 604 may comprise several independent subregions, such as a test line and a control line, or a test pad and a control pad, or more generally such as a test subregion and a control subregion.

In the cartridge 300, the test strip and the holder 308 are designed so that the detection region 604 faces the aperture(s) 312 of the holder 308, so that light from the light source(s) 402, 404 may reach the test strip. When there are several subregions, as previously disclosed, each subregion may face an aperture 312 in the holder 308.

The detection region 604 typically extends over the whole width of the test strip 600, notably for manufacturing reasons that will be disclosed later on.

In comparison with [Fig. 5], the detection region 604 corresponds to the test line 512a and the control line 514b of test strip 500a and to the colorimetric pad 512b, and the control pad 516b of test strip 500b. The migration pathway 606 corresponds to the sample pad 504d, the conjugate pad 506a, and the wicking pad 510a of test strip 500a and to the sample pad 504b, the migration membrane 508b and the wicking pad 510b.

The station 200, using in particular the analyzer 400, is configured to optically analyze the detection region 604 or at least a portion thereof. During the optical analysis, the light source 402, 404 illuminates the detection region 604 and the optical sensor 406 acquires optical data about the detection region 604, either via some light that has passed through the detection region 604 or via light emitted from the detection region 604 (for example when fluorophores are used).

However, during analysis, optical noise may be introduced by light leaks: stray light coming from the migration pathway, rather than the detection region, may reach the optical sensor 406, drowning out the relevant data in noise.

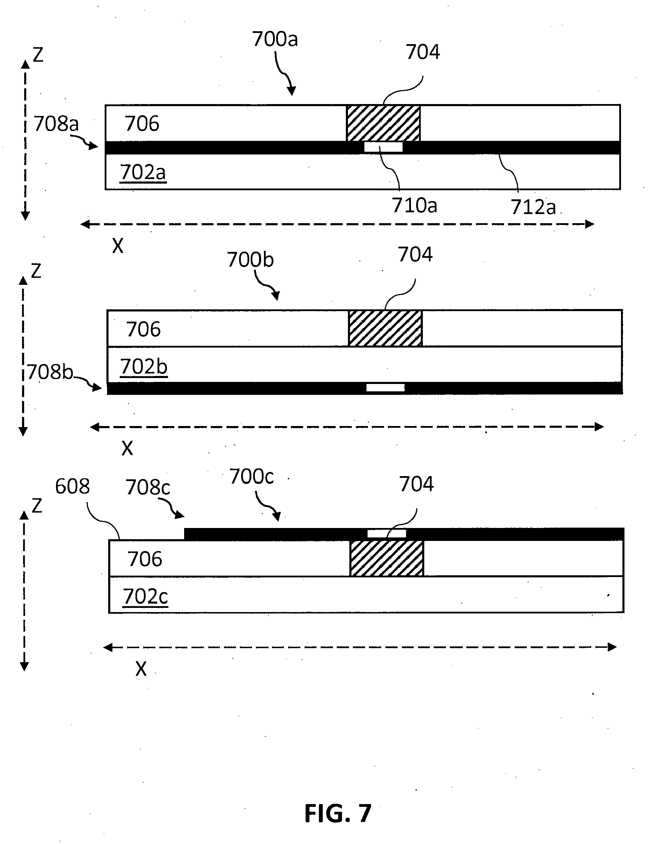

[Fig. 7] illustrates various embodiments of a test strip as presented in relation with [Fig. 6] with an optical mask to reduce or prevent light leaks. As described previously, the test strip comprises a detection region, with a reagent configured to react directly or indirectly with the substance of interest, and a migration pathway, configured to bring urine to the detection region. As it will be described in detail below, the test strip further comprises an optical mask which includes an opaque portion and a transparent portion. The opaque portion stops light, which does not go through the opaque portion, and the transparent portion lets light through and does not interfere with the optical analysis. The transparent portion faces at least a part of the detection region and the opaque portion is adjacent to the transparent portion. More precisely, the opaque portion covers at least the migration pathway in the vicinity of the detection region. This allows the optical analysis of the detection region to be carried out by the station with better results.

By transparent, it is meant that the light used for the optical analysis is not impaired by the transparent portion. In particular, the transparent portion may include a transparent material or simply nothing; the transparent portion is therefore defined by an aperture in the opaque portion.

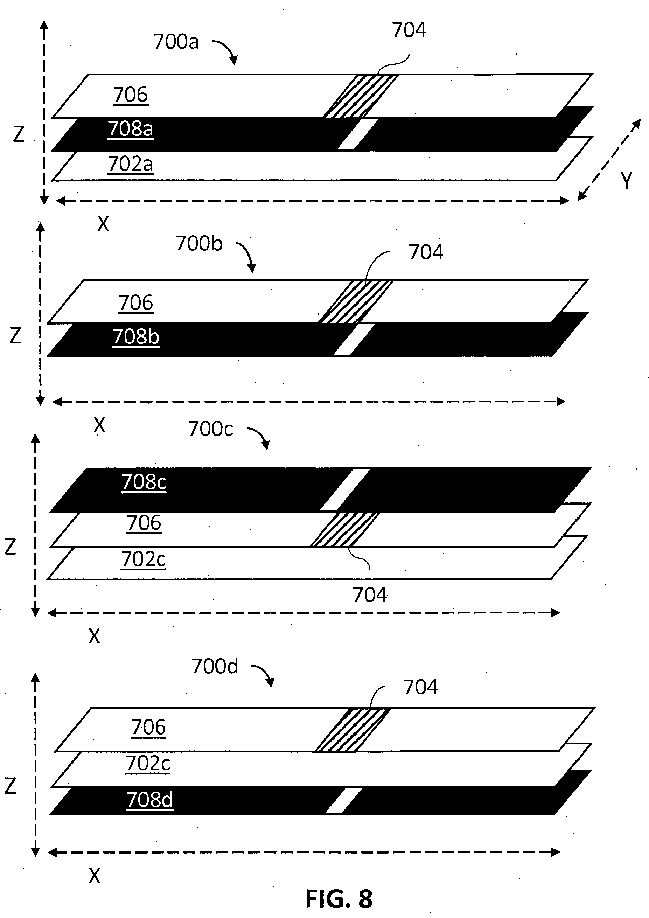

[Fig. 8] illustrates the embodiments of [Fig. 7] where the components are schematically represented in two dimensions. The thicknesses along the Z directions, the length along the X directions and the width along the Y directions are schematic.

Again, as described previously, the test strip may comprise a backing supporting the detection region and, if applicable, the migration pathway. To permit optical analysis in transmission, the backing is transparent (e.g., made of transparent plastic).

Test strip 700a of [Fig. 7] and [Fig. 8] illustrates a first embodiment. Test strip 700a comprises a backing 702a, a detection region 704 and a migration pathway 706. The backing 702a supports the migration pathway 706 and/or the detection 704. Test strip 700a further comprises an optical mask 708a, arranged between the backing 702a, and the detection region 704 and also arranged between the backing 702a and the migration pathway 706. By between it is meant between along Z direction. The optical mask 708a comprises an opaque portion 712a and a transparent portion 710a. Along the longitudinal direction X, the opaque portion 712a extends at least a one side of the detection region 704, notably on both sides of the detection region 704 as visible on Figure 7. In particular, the opaque portion 712a covers the migration pathway 706 in the vicinity of the detection region 704.The transparent portion 710a faces at least a part of the detection region 704 and the opaque portion may cover the rest of the detection region 704.

Test strip 700b of [Fig. 7] and [Fig. 8] illustrates a second embodiment. Test strip 700b is similar to test strip 700a except that the optical mask 708b is positioned under the backing 702b. This allows for a simplified adaptation of the test strip of Fig. 5 and Fig. 6, by merely adding the mask at the bottom of the backing.

Test strip 700c of [Fig. 7] and [Fig. 8] illustrates a third embodiment. Test strip 700c is similar to test strip 700a except that the optical mask 708c is positioned on top of the migration pathway 706 and on top of the detection region 705. This allows for a simplified adaptation of the test strip of Fig. 5 and Fig. 6, by merely adding the mask on top, providing that the top portion of the test strip is flat enough (more appropriate for colorimetric test strip for example than lateral flow). In addition, the depositing region 608 must be accessible. To that end, the optical mask 708c is not arranged on top of the depositing region 608, as visible on Figure 7.

For test strip 700a and 700b, the optical mask 708a, 708b may be made by printing the opaque portion on the backing (either side depending on the embodiment) and/or by adding an opaque tape on the backing.

Alternatively, the optical mask may comprise a transparent band onto which the opaque portions are added. The optical mask is then attached to the backing or the migration pathway.

In the present disclosure, by vicinity of the detection region, it is meant the immediate surrounding along the X direction of the detection region, or at least one side (e.g., both sides) along the X direction of the detection region. For example, this means 1mm or 2mm on at least one side of the detection region; therefore, the first 1mm or 2mm of the migration pathway that connects to the detection portion is covered by the opaque portion. In other words, vicinity of the detection region means the migration pathway that appears as adjacent to the detection region, when looked from the X direction. In the present disclosure, by facing or covers, it is meant along the Z direction. As the strip extends essentially along the X direction and is made of different components stacked on the Z directions, the terms "facing" or "covering" are appropriate.

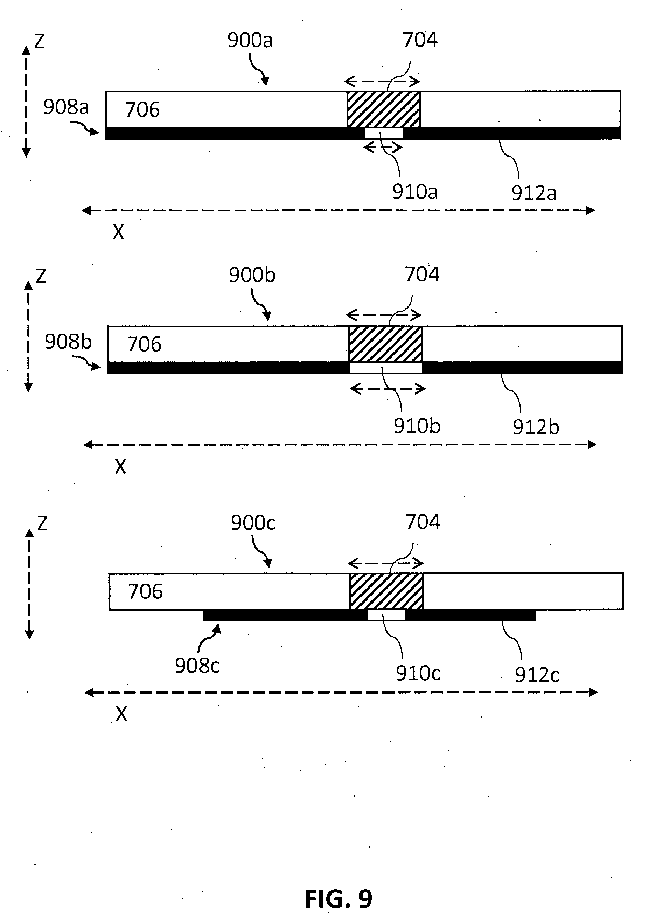

[Fig. 9] illustrates in schematic way different variations, all applicable to the test strips of [Fig. 7] and [Fig. 8]. In [Fig. 9], only the optical mask, the migration pathway 706 and the detection region 704 are represented (in a schematic manner).

In a variation, test strip 900a has an optical mask 908a that has a transparent portion 910a with a dimension smaller along the X direction which is smaller than that of the detection region 704. Therefore, the optical mask 908a has an opaque portion 912a that also covers a portion of the detection region 704 (i.e., the portion of the detection region 704 that does not face the transparent portion 910a). The opaque portion 912a also covers the migration pathway 706 in the vicinity of the detection region 704. This creates an efficient optical mask that guides light only through the detection region 704. This also allows to manufacture a test strip with fewer constraints about the size of the detection region 704, as the optical mask 908a may be clearly delimited on the test strip.

In a variation, test strip 900b has an optical mask 908b that has a transparent portion 910b with a dimension along the X direction which is the same than that of the detection region 704. More particularly, the transparent portion 910b faces and matches the detection region 704 in the Z direction. Therefore, the optical mask 908b has an opaque portion 912b that does not cover a portion of the detection region 704. However, the opaque portion 912b covers the migration pathway 706 in the vicinity of the detection region 704. This creates an efficient optical mask that guides light only through the detection region. This is appropriate when the detection region has the smallest acceptable size and the manufacturer does not wish to lose some of its region because of the optical mask.

In a variation, similar to either the test strip 900a or the test strip 900b, test strip 900c has an optical mask 908c hat has an opaque portion 912c which covers a portion only of the migration pathway 706, such as at least 90%. Indeed, light leaks are likely to occur in the vicinity of the detection region 704; far therefrom, the test strip is likely to be hidden by the holder 308 and therefore no light will reach the portion of the migration pathway 706 that is not covered by the opaque portion 912c of the optical mask. However, as illustrated on test strip 900a, 900b, 100% of the migration pathway 706 may be covered by the optical mask.

The transparent portion may be located, along the X direction, strictly between the edges of the detection region 704 (as illustrated in test strip 900a). This means that on both sides along the X direction of the transparent portion, there is the opaque portion that covers a portion of the detection region. However, depending on the arrangement of the light source and the light sensor, the transparent portion may be located at one extremity of the detection region 704, so that the opaque portion on one side along the X direction covers the migration pathway 706 and the opaque portion on the other side along the X direction faces a portion of the detection region 704.

In an embodiment, not illustrated, the transparent portion also extends in the vicinity of the detection region (only in the vicinity though, on at least one side along the X direction). This provides a version of the optical mask with degraded results compared to the previous embodiments but still more efficient in terms of light leaks than without the optical mask. Such embodiment may present an advantage when the repeatability of the position of the test strip in the cartridge is hard to achieve. A larger transparent portion brings more tolerance as regards said position. By vicinity here, it is meant less than 1mm in an example.

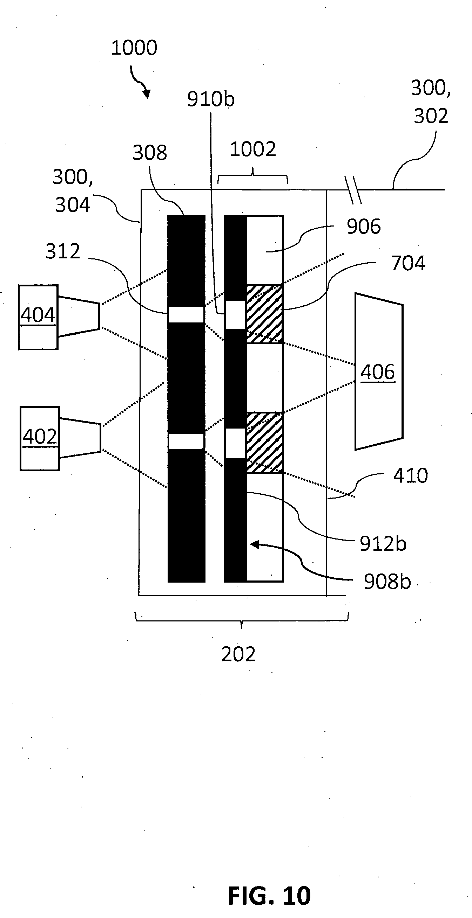

[Fig. 10] illustrates a representation 1000 of the rational of the optical mask using a test strip 1002 similar to test strip 900a but with a detection region 704 made of two subregions and thus an optical mask 908b with two transparent portions 910b facing said subregions (and the opaque portion 912b extending between the two subregions and covering the migration pathway in the vicinity of the detection region 704). From the left to the right, there is: the light sources 402, 404, the cylindrical portion 304 of the rotatable support 300 (which is transparent to light), the holder 308 with its apertures 312, the test strips with its optical mask, migration pathway and detection region, the lid 410, and the optical sensor 406.

The oblique dashed lines illustrate different beams of light going through the cartridge 202. A detailed light pathway will be explained for one light source, but the reasoning is the same for all. The light beam first exits the light source 404 towards the cartridge 202. Because of the aperture 312, only a fraction of that beam goes through the holder 308. At the exit of the aperture 312 diffraction occurs and a new light beam is generated. This time, because of the opaque portion 912b of optical mask 908b, only the detection region 704 is reached by the new light beam. In particular, the migration pathway 406 around the detection region 910b in the X direction does not receive light from the new light beam. Without the optical mask 908b, the light beam of the aperture 312 would reach the migration pathway 906 as well, hereby generating noise in the optical sensor 406 (i.e., light going through the migration pathway 906). This time again, because of the transparent portion 910b, a new light beam is generated but the light that reaches the optical sensor 406 has only gone through the detection region 704.

Indeed, without the optical mask, light that would come from the migration pathway 706 would not be colored as light coming from the detection region 704 (the migration pathway is usually white, while the detection region 704 is configured to change its colors based on a chemical reaction with urine). Therefore, an optical dilution may occur when light coming from the migration pathway 706 and light coming from the detection region 704 reach the optical sensor 406. The optical dilution meddles with the sensitivity threshold of the optical sensor 406, which may be low, and therefore impairs the quality of the physical/chemical property analysis. With the optical mask 908b, only colored light coming from the detection region 704 reaches the optical sensor 406.

Similar results are obtained with the test strips 700a, 700b, 700c and their variations 900a, 900b, 900c. In particular, the transparent region may be designed so that the diffraction occurring therein creates a light beam that only goes through the detection region. In that respect, the embodiment of test strip 900a presents such an advantage, by having a transparent region 910a smaller than the detection region (or their respective subregion).

[Fig. 10] shows an implementation where the analysis works in light transmission. However, the same rationale applies when the analysis works in reflection (i.e., light source and optical sensor on the same side). Similarly, the implementation works when the light comes directly from the detection region (e.g., fluorophores that emit light when excited with a certain wavelength).

The following dimensions are given as examples: along the X direction, the length of the test strip may be comprised between 10mm and 15mm; along the Y direction, the width of the test strip may be comprised between 1.0mm and 1.5mm.Along the X direction, the cumulated length of the detection region may be less than 5mm, even less than 3mm or 2mm. Along the X direction, the length of the transparent portion may be less than 2 mm, for example less than 1 mm, for example between 0.2mm and 0.5mm (for example 0.3mm). When they are two subregions for the detection region, the distance between the two transparent portions may be comprised between 1 and 3mm.

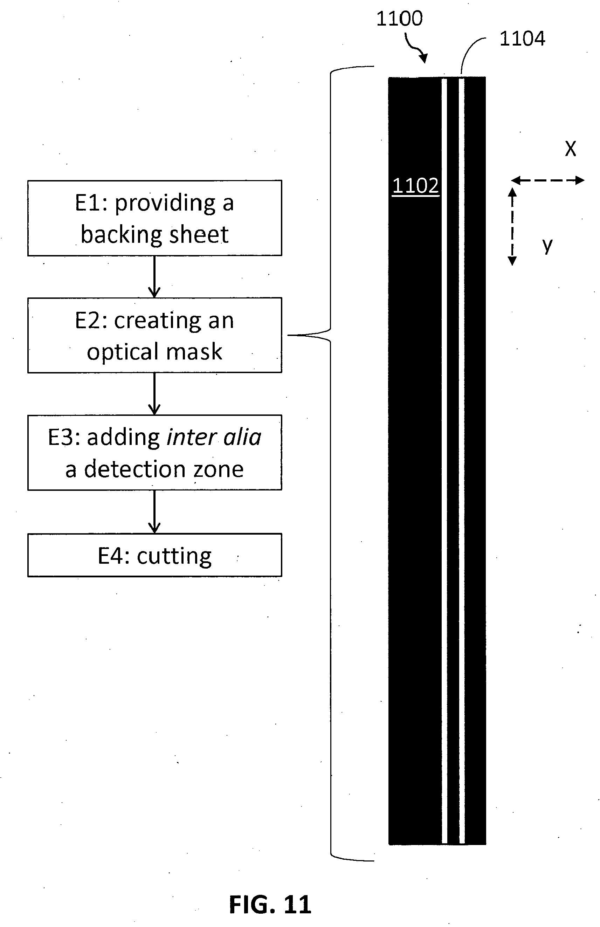

[Fig. 11] shows a process to manufacture test strips according to embodiments of the disclosure, along with an illustration of a face of a backing with an optical mask applied thereon. To manufacture the test strips disclosed, it is more convenient to prepare a sheet on which a plurality of test strips are generated side by side, adjacent to one another, by typically providing the different components forming the test strip (deposition region, detection region, etc.) and thus cutting said sheet.

For example, a backing sheet 1100 may be provided (step E1). Onto a face of that backing sheet, the optical mask is created by printing the opaque zone 1102 and by letting the backing sheet 1100 as is to generate a transparent zone 1104 (step E2). The transparent zone 1104 comprises one or two parallel lines (corresponding to the detection region of the two detection subregions), depending on the embodiment.

On the other face of the backing sheet 1100 (embodiment of test strip 708b), or on optical mask (embodiment of test strip 700a), the migration pathway, the depositing region, the collection region and the detection region are added (step E3 and backing sheet 1100). This creates a sheet comprising a plurality of test strips parallel to one another and still attached. Therefore, a cutting step (step E4), along the longitudinal direction (and therefore perpendicular to the parallel lines of the transparent zone 1104) is provided to create the test strips. This process simplifies the manufacturing operations as the transparent portion are quickly generated for a plurality of test strips. A consequent of such process is that the transparent portion of the optical mask extends along the whole width of the test strip.

The test strips disclosed here may be used with the cartridge previously discloses and the urine analysis device. They provide a cost efficient, manufacturing efficient, easily adaptable, and mass-production suitable solution to improve the quality of the optical analysis.

## Claims

1. A test strip (700a, 700b, 700c) for an optical analysis of a substance, the test strip comprising:
- a detection region (704) comprising a reagent to react directly or indirectly with the substance,
- an optical mask (708a, 708b, 708c) including a transparent portion (710a) facing at least a part of the detection region (604, 704) and an opaque portion (712a) adjacent to the transparent portion (712a).

2. The test strip (700a, 700b, 700c) according to claim 1, comprising a migration pathway (706) in contact with the detection region (704) and configured to receive and migrate the substance, wherein the optical mask covers at least partially the migration pathway.

3. The test strip according to claim 2, wherein the optical mask covers more than 90% of the migration pathway.

4. The test strip according to any of claims 1-3, wherein the test strip extends along a longitudinal direction (X) and the opaque portion extends on at least one side of the transparent region along the longitudinal direction (X).

5. The test strip according to any of claims 1-4, wherein the transparent portion is smaller than the detection region.

6. The test strip according to any of claims 1-5, wherein the detection region includes at least two subregions spaced apart from each other, and wherein the opaque portion extends therebetween.

7. The test strip according to any of claims 1-6, further comprising a backing (502a, 502b, 502c, 602) supporting the detection region, wherein the optical mask is positioned between the detection region and the backing.

8. The test strip according to any of claims 1-7, further comprising a backing supporting the detection region, wherein the backing is positioned between the optical mask and the detection region.

9. The test strip according to any of claims 7-8, wherein the optical mask is provided on a face on the backing.

10. The test strip according to any of claim 1-9, further comprising a backing supporting the detection region, wherein the detection region is positioned between the optical mask and the backing.

11. The test strip according to any of claims 1-10, wherein a length of the test strip is less than 20mm, and/or a width of the test strip is less than 5mm and/or a cumulated length of the detection region is less than 5mm.

12. The test strip according to any of claims 1-11, wherein the transparent portion of the optical mask extends along a whole width of the test strip.

13. A cartridge (202), comprising a plurality of chambers (310) arranged next to one another in a right circular cylinder shape of at least 80% of a circle with a diameter comprised between 3 and 10 cm, wherein each chambers comprises a test strip according to any of claims 1 to 12.

14. A urine analysis device (100) for urine analysis comprising:
- the cartridge (202) according to claim 13,
- a station (200), configured to be positioned on a wall of a toilet bowl (106), the station comprising:
. a case (204) comprising an annular housing (212) in which the cartridge is at least partially received and is rotatably mounted therein around a rotation axis (A),
. an injector (412), configured to inject urine on the test strip,
. an analyzer (400) with a light source (402, 404) and a light sensor (406), configured to emit and receive light, and to detect a change of a property of the detection zone of the test strip.

15. A process to manufacture a test strip according to any of claims 1-12, comprising:
- (E1) providing a backing sheet (1100),
- (E2) generating an optical mask by generating on a face of the backing sheet an opaque zone (1102) and a transparent zone (1104),
- (E4) cutting the backing sheet to generate a test strip.
